# FASCICULE DE BREVET EUROPEEN

(11) **EP 3 011 994 B1**
(45) Date de publication et mention de la délivrance du brevet: **10.10.2018**
(21) Numéro de dépôt: 15306281.5
(22) Date de dépôt: 10.08.2015
(51) Int. Cl.: A61M 39/10, A61M 39/26

(54) **CONNECTEUR LUER FEMELLE**
WEIBLICHER LUER-LOCK
FEMALE LUER CONNECTOR

(43) Date de publication de la demande: 27.04.2016
(73) Titulaire: Cair L. G. L., 69380 Lissieu (FR)
(72) Inventeur: LOPEZ, Georges Antoine, 69130 ECULLY (FR); DELORME, Patrick, 69630 CHAPONOST (FR)
(74) Mandataire: Cabinet Laurent & Charras

(56) Documents cités:
- US-A- 6 063 062
- US-A- 6 068 011
- US-A1- 2002 013 556
- US-A1- 2003 098 430
- US-A1- 2007 218 757

## Description

### DOMAINE TECHNIQUE

La présente invention concerne un site d'injection sans aiguille sous la forme d'un connecteur luer femelle destiné à un usage médical. L'invention trouve une application particulièrement avantageuse pour maintenir une seringue dans un connecteur luer femelle qui n'est pas du type luer lock.

### ART ANTERIEUR

Il existe des sites d'injection avec aiguille dans lesquels un connecteur comporte une chambre centrale débouchant sur deux extrémités. Une première extrémité du connecteur est pourvue d'un raccord configuré pour recevoir une tubulure et une seconde extrémité, opposée à la première extrémité, comporte une membrane. Pour connecter une seringue sur ce connecteur, une aiguille agencée à l'extrémité de la seringue perce et traverse la membrane afin d'accéder dans la chambre. Une communication hydraulique est ainsi réalisée entre la seringue portant l'aiguille et le raccord.

Contrairement aux sites d'injection avec aiguille, les sites d'injection sans aiguille ont pour objectif de connecter une seringue avec une tubulure sans que la seringue ne porte d'aiguille. A cet effet, il existe des connecteurs du type luer mâle et femelle. Un luer femelle est fixé sur la tubulure reliée au patient et un luer mâle est formé à l'extrémité de la seringue. Le luer femelle comporte une chambre centrale débouchant sur deux extrémités. La première extrémité du luer femelle est pourvue d'un raccord configuré pour recevoir une tubulure et la seconde extrémité, opposée à la première extrémité, comporte une embouchure configurée pour recevoir le luer mâle de la seringue. Lorsqu'aucune connexion n'est établie, l'embouchure est généralement obturée par un bouchon. Pour connecter le luer mâle de la seringue sur le luer femelle, le bouchon est retiré et le luer mâle est inséré dans l'embouchure pour entrer en communication hydraulique avec la chambre et le raccord. Dans la présente demande, les connecteurs luers femelles soit décrits par leurs caractéristiques propres et ne correspondent pas forcément à la norme ISO 80369-7.

Une autre solution, décrite dans la demande de brevet internationale N° WO 2014/023921 de la demanderesse, consiste à utiliser une clé amovible à la place du bouchon pour assurer l'obturation de la connexion. A cet effet, la clé présente une portion formant ressort disposée dans la chambre et une tête s'étendant dans l'embouchure. La portion formant ressort est destinée à être compressée dans la chambre lorsque le luer mâle de la seringue pénètre dans l'embouchure et appuie sur la tête de la clé de sorte à établir une communication hydraulique entre la seringue et le raccord. Les documents US 6,068,011, US 2007/0218757 et US 2003/0098430 décrivent un connecteur luer femelle présentant une embouchure positionnée au-dessus d'une chambre centrale. Un angle saillant est formé entre la portion tronconique terminale de la chambre centrale et la paroi tronconique de l'embouchure. Cet angle délimite l'embouchure de la chambre centrale et vice-versa.

Pour maintenir le luer mâle en position dans l'embouchure du luer femelle, il est connu d'utiliser une fixation réalisée au moyen d'un filetage coopérant avec un filetage correspondant du luer femelle. De tels connecteurs sont appelés « luer lock ». D'autres connecteurs luers mâles, plus simples, ne comportent pas de moyens de fixation et sont maintenus dans le connecteur luer femelle uniquement par la force de friction générée par l'appui du luer mâle sur l'embouchure.

Les seringues comportaient jusqu'alors trois pièces : un corps, un piston et une pièce en élastomère destinée à assurer une étanchéité entre le corps et le piston. Des nouvelles seringues comportent uniquement deux pièces : un corps et un piston. Pour ce faire, le corps de ces nouvelles seringues est fabriqué en un plastique auto-lubrifié permettant d'assurer une étanchéité entre le corps et le piston. Une telle matière est, par exemple, un Polypropylène. Cette matière diminue la force de friction du luer mâle de la seringue dans l'embouchure. Ainsi, lorsqu'un nouveau luer mâle est introduit dans l'embouchure, la force de rappel de la tête de la clé dans l'embouchure est supérieure à la force de friction générée par l'appui du luer mâle sur l'embouchure. Le luer mâle est donc éjecté de l'embouchure si l'opérateur ne maintient pas une pression suffisante sur le luer mâle.

Lorsqu'un opérateur utilise une nouvelle seringue avec un connecteur luer femelle, il doit maintenir d'une main le site d'injection sans aiguille et il utilise l'autre main pour presser le piston de la seringue avec un geste maîtrisé de sorte à contrôler le débit de fluide à administrer. Les gestes de l'opérateur sont donc très complexes à exécuter ce qui entraine un risque de contamination par une maladresse de l'opérateur.

Pour résoudre ce problème, certains opérateurs exercent une pression très importante sur le connecteur luer mâle inséré dans l'embouchure de sorte à maintenir en compression le connecteur luer mâle dans l'embouchure. Même avec cette solution, il arrive cependant que le connecteur luer mâle soit éjecté de manière inopinée.

Le document US 6,063,062 décrit un connecteur luer femelle comportant une embouchure munie d'un ergot déformable. La force de friction générée par l'ergot déformable n'est pas suffisante pour maintenir le connecteur luer mâle dans l'embouchure.

Le document US 2002/013556 décrit un connecteur luer femelle dont l'embouchure tronconique présente des nervures qui peuvent se déformer suite à l'insertion du connecteur lueur mâle. La solution proposée ne permet pas de maintenir le luer male dans l'embouchure de manière efficace dans la mesure où les moyens de retenue sont longitudinaux et au contact de la seule surface de la paroi du connecteur luer mâle.

Le problème technique de l'invention consiste donc à maintenir un connecteur luer mâle dans l'embouchure d'un connecteur luer femelle comportant une clé, et plus généralement un site d'injection sans aiguille.

### EXPOSE DE L'INVENTION

La présente invention vise à résoudre ce problème technique en utilisant des moyens de retenue faisant saillie d'une paroi interne de l'embouchure

A cet effet, selon un premier aspect, l'invention concerne un connecteur luer femelle comportant :
- un capot présentant :
   - une première extrémité apte à être connectée à un patient,
   - une seconde extrémité formée par une embouchure du capot et configurée pour recevoir un connecteur luer mâle, et
   - une chambre centrale débouchant d'une part sur le raccord et d'autre part sur l'embouchure, et
- une clé présentant une portion formant ressort disposée dans la chambre et une tête s'étendant dans l'embouchure,
- la portion formant ressort étant destinée à être compressée dans la chambre lorsqu'un connecteur luer mâle pénètre dans l'embouchure et appui sur la tête de la clé de sorte à établir une communication hydraulique entre le connecteur lueur mâle et le raccord; L'invention se caractérise en ce que la paroi interne de l'embouchure comporte des moyens de retenue présentant un angle saillant qui s'étend radialement en direction du centre de l'embouchure de sorte à pénétrer dans un connecteur luer mâle pour que la force de friction générée par l'appui d'un connecteur luer mâle sur l'embouchure soit supérieure à la force de rappel de la tête de la clé dans l'embouchure.

L'invention permet ainsi de maintenir efficacement un connecteur luer mâle dans l'embouchure sans que l'opérateur exerce une force importante sur le connecteur luer mâle. Pour résoudre le problème technique de l'invention, l'homme du métier du domaine des connecteurs luers aurait recherché une solution du type luer lock. Or, la solution non intuitive de l'invention est plus simple à mettre en oeuvre et ne nécessite pas de modifier le connecteur luer mâle généralement disposé sur une seringue.

Les connecteurs luer mâle équipent de manière connue les seringues et plus généralement toutes les lignes de perfusion à destination d'un patient.

Selon un mode de réalisation, l'embouchure et les moyens de retenue sont réalisés en polycarbonate. Ce mode de réalisation permet à l'embouchure et aux moyens de retenue de présenter un module d'élasticité supérieure au module d'élasticité d'un connecteur luer mâle réalisé en Polypropylène. La force de friction générée par l'appui du connecteur luer mâle sur l'embouchure est ainsi augmentée.

Selon un mode de réalisation, les moyens de retenue présentent un angle saillant qui s'étend radialement en direction du centre de l'embouchure. Ce mode de réalisation permet aux moyens de retenue de pénétrer au coeur du matériau formant le connecteur luer mâle même si le connecteur luer mâle est revêtu d'une matière externe auto-lubrifiée. La force de friction générée par l'appui du connecteur luer mâle sur l'embouchure est ainsi augmentée.

Selon un mode de réalisation, les moyens de retenue présentent un profil triangulaire, une première face sensiblement plane étant configurée pour permettre à un connecteur luer mâle de descendre dans l'embouchure et une deuxième face sensiblement plane étant configurée pour permettre à la tête de la clé de remonter dans l'embouchure. Ce mode de réalisation permet de faciliter l'insertion et le retrait du connecteur luer mâle en position dans l'embouchure.

Selon un mode de réalisation, les moyens de retenue présentent un profil triangulaire isocèle, la première face présentant une longueur supérieure à une longueur de la deuxième face.

Selon un mode de réalisation, le capot présentant un épaulement interne entre le volume de la chambre et celui de l'embouchure, les moyens de retenue sont disposés à proximité de l'épaulement. Ce mode de réalisation permet d'effectuer le maintien du connecteur luer mâle uniquement lorsque celui-ci a pénétré entièrement dans l'embouchure. L'opérateur peut ainsi ressentir que la force de rappel de la clé est annulée lorsqu'il a atteint la position requise pour une communication hydraulique entre le connecteur luer mâle et le raccord.

Selon un mode de réalisation, les moyens de retenue présentent une hauteur comprise entre 10 et 50 µm et une longueur comprise entre 30 et 80 µm. Ces dimensions particulières permettent à un connecteur luer mâle de descendre dans l'embouchure et à la tête de la clé de remonter dans l'embouchure.

Selon un mode de réalisation, le connecteur luer femelle comporte une aiguille agencée dans la chambre dont le volume interne est connecté au raccord, l'aiguille étant destinée à traverser une paroi terminale de la tête de la clé lorsque la clé est comprimée dans la chambre par un connecteur luer mâle. Ce mode de réalisation permet à l'aiguille de pénétrer dans le volume interne du connecteur luer mâle lorsque le connecteur luer mâle est inséré dans le connecteur luer femelle. Des ouvertures ménagées au niveau de l'extrémité de l'aiguille permettent au fluide présent dans le connecteur luer mâle de pénétrer dans l'aiguille et ainsi d'atteindre le raccord.

Selon un mode de réalisation, le connecteur luer femelle comporte une bague agencée autour de la tête de la clé et destinée à guider les déplacements de la clé de manière rectiligne selon une longueur du capot. Ce mode de réalisation permet de guider efficacement les déplacements de la clé lors de l'insertion ou du retrait du connecteur luer mâle dans le connecteur luer femelle.

Selon un deuxième aspect, l'invention concerne un ensemble de raccordement à usage médicale comportant un connecteur luer mâle et un connecteur luer femelle (10) selon le premier aspect de l'invention.

### DESCRIPTION SOMMAIRE DES FIGURES

La manière de réaliser l'invention ainsi que les avantages qui en découlent, ressortiront bien du mode de réalisation qui suit, donné à titre indicatif mais non limitatif, à l'appui des figures annexées dans lesquelles les figures 1 à 4 représentent :
- Figure 1 : une vue en perspective d'un connecteur luer femelle selon un mode de réalisation de l'invention ;
- Figure 2 : une vue en coupe d'un connecteur luer mâle distant du connecteur de la Figure 1 ;
- Figure 3 : une vue en coupe des connecteurs de la Figure 2 lorsque le connecteur luer mâle est inséré dans le connecteur luer femelle ;
- Figure 4 : un agrandissement de la Figure 2 au niveau d'une embouchure du connecteur luer femelle destinée à recevoir le connecteur luer mâle ; et
- Figure 5 : un agrandissement de la Figure 3 au niveau de l'embouche du connecteur luer femelle coopérant avec le connecteur luer mâle.

### DESCRIPTION DETAILLEE DE L'INVENTION

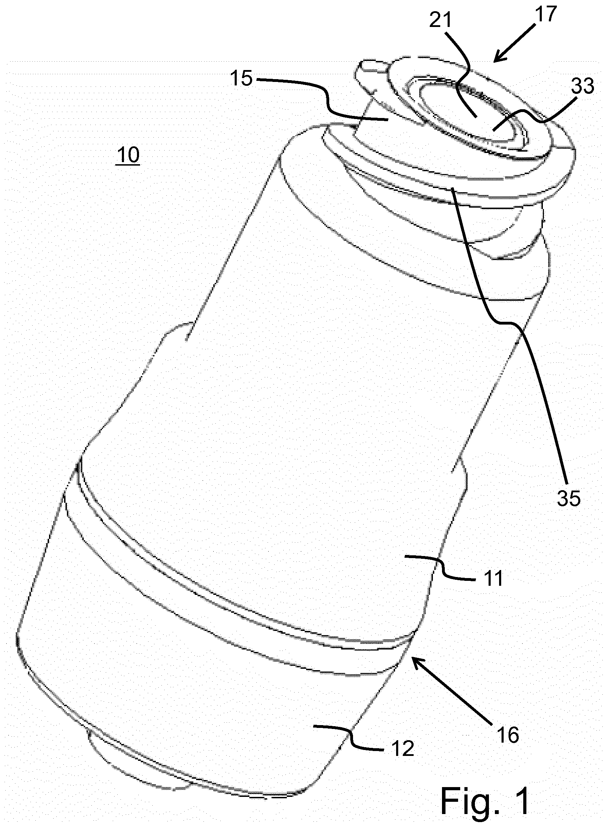

La Figure 1 illustre un connecteur luer femelle **10** (ou luer femelle) comportant un capot **11** dont une première extrémité **16** est connectée à un raccord **12** configuré pour recevoir une tubulure. Une seconde extrémité **17,** opposée à la première extrémité **16,** comporte un rétrécissement du volume délimité par le capot **11** formant une embouchure **15.** Le luer femelle **10** comporte un filetage **35** réalisé sur une face cylindrique externe de l'embouchure **15** de sorte à pouvoir connecter un connecteur luer mâle **20** (ou luer mâle) du type luer lock. Le connecteur luer femelle **10** illustré sur la Figure 1 permet de connecter un luer mâle **20** dans l'embouchure **15** avec ou sans fixation. L'embouchure **15** est obturée par une clé **21.**

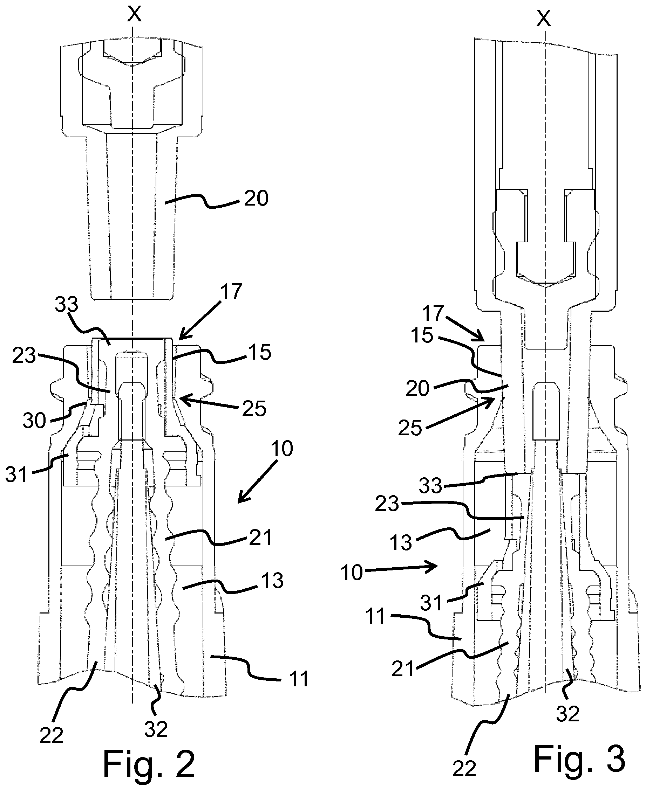

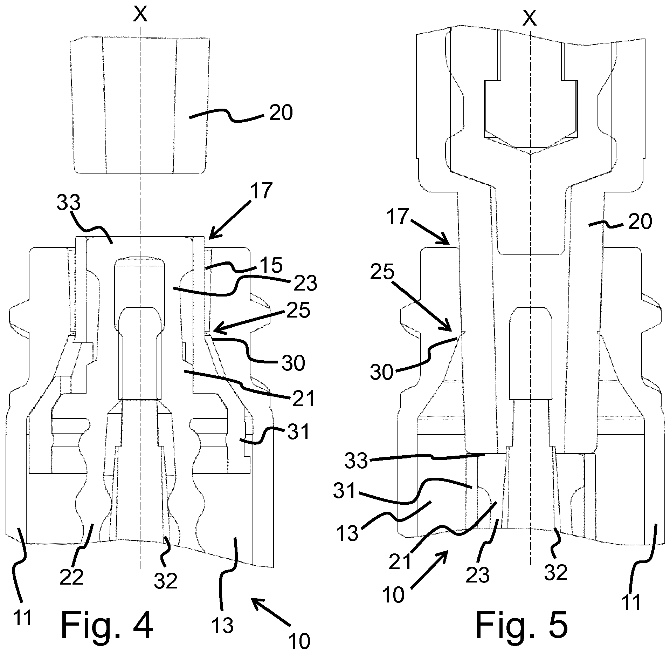

Le détail de l'agencement de cette clé **21** dans le capot **11** est illustré sur les vues en coupe des Figures 2 à 5. Le capot **11** permet de délimiter une chambre interne **13** débouchant d'une part sur le raccord **12** et d'autre part sur l'embouchure **15.** La position de la clé **21** dans le luer femelle **10** dépend de la présence ou non du luer mâle **20.** Sur les Figures 2 et 4, le luer mâle **20** est distant du luer femelle **10** alors que sur les Figures 3 et 5, le luer mâle **20** est inséré dans le luer femelle **10.**

Lorsque le luer mâle **20** est distant du luer femelle **10,** la clé **21** présente une portion formant ressort **22** s'étendant dans la chambre **13** et une tête **23** s'étendant dans l'embouchure **15.** Une aiguille **32** est agencée dans la chambre **13** à l'intérieur du volume de la clé **21.** Le volume interne de l'aiguille **32** est en communication hydraulique avec le raccord **12.** Une bague **31** est agencée autour de la tête **23** de la clé **21** et bute contre une paroi tronconique du capot **11** reliant le plus important volume de la chambre **13** à l'embouchure **15.** Dans cette position, la clé **21** forme une barrière étanche autour de l'aiguille **32,** isolant ainsi le patient des bactéries externes.

Lorsqu'un luer mâle **20** pénètre dans le luer femelle **10,** il est dirigé dans l'embouchure **15** selon un axe **X** longitudinal du capot **11.** Pour réaliser le guidage du luer mâle **20,** l'embouchure **15** peut présenter une paroi interne cylindrique ou tronconique, par exemple avec un angle de 6%. Le luer mâle **20** bute alors contre la tête **23** de la clé **21** et comprime la portion formant ressort **22** dans la chambre **13.**

Lorsque l'aiguille **32** bute contre une paroi terminale **33** de la tête **23,** l'aiguille **32** ouvre une micro-ouverture ménagée sur la paroi terminale **33** et traverse la paroi terminale **33.** La micro-ouverture permet de garantir l'étanchéité de la paroi terminale **33** de la tête **23** lorsque le luer mâle **20** est distant du luer femelle **10.** Les capacités de déformation de cette paroi terminale **33** permettent d'autoriser le passage de l'aiguille **32** sans détruire l'étanchéité future du luer femelle **10.** Lors de la compression de la portion formant ressort **22** de la clé **21,** la bague **31** guide les déplacements de la tête **23** de la clé **21.**

Lorsque le luer mâle **20** est complètement inséré dans le luer femelle **10,** tel qu'illustré sur les Figures 3 et 5, une extrémité de l'aiguille **32** est située dans le volume du luer mâle **20.** L'extrémité de l'aiguille **32** comporte un chat de sorte à établir une communication hydraulique entre le luer mâle **20** et le raccord **12** par l'intermédiaire du volume interne de l'aiguille **32.** Ce mode de connexion entre les connecteurs luers mâle **20** et femelle **10** illustre uniquement un mode de réalisation non limitatif de l'invention. En variante, le luer femelle **10** peut être dépourvu de bague **31** ou d'aiguille **32** sans changer l'invention.

L'embouchure **15** comporte, en outre, des moyens de retenue **25** faisant saillit de la paroi interne de l'embouchure **15.** Les moyens de retenue **25** sont configurés de sorte que la force de friction générée par l'appui du luer mâle **20** sur l'embouchure **15** est supérieure à la force de rappel de la tête **23** de la clé **21** dans l'embouchure **15.**

Les moyens de retenu **25** peuvent prendre toutes les formes imaginables, par exemple un bourrelet continue ou discontinu sur tout ou partie de la périphérie interne de l'embouchure **15,** une hélice, des ergots... De préférence, les moyens de retenue **25** présentent un angle saillant qui s'étend radialement en direction du centre de l'embouchure **15,** c'est-à-dire en direction de l'axe central **X.** Dans le mode de réalisation des Figures 2 à 5, les moyens de retenue **25** sont réalisés pas un ergot annulaire présentant un profil triangulaire. La face annulaire supérieure de l'ergot permet au luer mâle **20** de descendre dans l'embouchure **15** et la face annulaire inférieure de l'ergot permet à la clé **21** et à la bague **31** de remonter dans l'embouchure **15.** En variante, le profil peut être triangulaire isocèle avec la face supérieure plus grande que la face inférieure. Par exemple, les moyens de retenu **25** présentent une hauteur comprise entre 10 et 50 µm et une largeur comprise entre 30 et 80 µm.

La position des moyens de retenu **25** dans l'embouchure **15** est également un paramètre qui peut varier sans changer l'invention. De préférence, les moyens de retenu **25** sont disposés au niveau d'un épaulement **30** entre le volume de la chambre **13** et celui de l'embouchure **15.** La matière des moyens de retenu **25** est déterminée de sorte à présenter un module d'élasticité supérieur à celui de luer mâle **20.** Par exemple, lorsqu'une seringue est réalisée en Polypropylène avec un module d'élasticité sensiblement égal à 1250 mPa. Les moyens de retenu **25** peuvent ainsi être réalisés en Polycarbonate avec un module d'élasticité sensiblement égal à 2350 mPa. Les modules d'élasticité sont déterminés selon la norme ISO 527.

L'invention permet ainsi de maintenir efficacement un luer mâle **20** d'une seringue dans un luer femelle **10** sans utiliser un connecteur luer lock.

## Revendications

1. Connecteur luer femelle (10) comportant :
- un capot (11) présentant :
- une première extrémité (16) apte à être connectée à un patient,
- une seconde extrémité (17) comportant un rétrécissement de volume formant une embouchure (15) présentant une paroi interne cylindrique ou tronconique et configurée pour recevoir un connecteur luer mâle (20), et
- une chambre (13) centrale débouchant d'une part sur le raccord (12) et d'autre part sur l'embouchure (15), et
- une clé (21) présentant une portion formant ressort (22) disposée dans la chambre (13) et une tête (23) s'étendant dans l'embouchure (15),
- la portion formant ressort (22) étant destinée à être compressée dans la chambre (13) lorsqu'un connecteur luer mâle (20) pénètre dans l'embouchure (15) et appui sur la tête (23) de la clé (21) de sorte à établir une communication hydraulique entre le connecteur lueur mâle (20) et le raccord (12),
***caractérisé en ce que*** ladite paroi interne de l'embouchure (15) comporte des moyens de retenue (25) présentent un angle saillant qui s'étend radialement en direction du centre de l'embouchure (15) de sorte à pénétrer dans un connecteur luer mâle (20) pour que la force de friction générée par l'appui d'un connecteur luer mâle (20) sur l'embouchure (15) soit supérieure à la force de rappel de la tête (23) de la clé (21) dans l'embouchure (15).

2. Connecteur luer femelle (10) selon la revendication 1, ***caractérisé en ce que*** l'embouchure (15) et les moyens de retenue (25) sont réalisés en polycarbonate.

3. Connecteur luer femelle (10) selon l'une des revendications 1 à 2, ***caractérisé en ce que*** les moyens de retenue (25) présentent un profil triangulaire, une première face sensiblement plane étant configurée pour permettre à un connecteur luer mâle (20) de descendre dans l'embouchure (15) et une deuxième face sensiblement plane étant configurée pour permettre à la tête (23) de la clé (21) de remonter dans l'embouchure (15).

4. Connecteur luer femelle (10) selon la revendication 3, ***caractérisé en ce que*** les moyens de retenue (25) présentent un profil triangulaire isocèle, la première face présentant une longueur supérieure à une longueur de la deuxième face.

5. Connecteur luer femelle (10) selon l'une des revendications 1 à 4, ***caractérisé en ce* que**, le capot (11) présentant un épaulement (30) interne entre le volume de la chambre (13) et celui de l'embouchure (15), les moyens de retenue (25) sont disposés à proximité de l'épaulement (30).

6. Connecteur luer femelle (10) selon l'une des revendications 1 à 4, ***caractérisé en ce que*** les moyens de retenue (25) présentent une hauteur comprise entre 10 et 50 µm et une longueur comprise entre 30 et 80 µm.

7. Connecteur luer femelle (10) selon l'une des revendications 1 à 6, ***caractérisé en ce* qu'**il comporte une aiguille (32) agencée dans la chambre (13) dont le volume interne est connecté au raccord (12), l'aiguille (32) étant destinée à traverser une micro-ouverture d'une paroi terminale (33) de la tête (23) de la clé (21) lorsque la clé (21) est comprimée dans la chambre (13) par un connecteur luer mâle (20).

8. Connecteur luer femelle (10) selon l'une des revendications 1 à 7, ***caractérisé en ce* qu'**il comporte une bague (31) agencée autour de la tête (23) de la clé (21) et destinée à guider les déplacements de la clé (21) de manière rectiligne selon une longueur du capot (11).

## Patentansprüche

1. Luer-Buchse (10) mit
- einer Abdeckung (11), die aufweist:
- ein erstes Endstück (16), das mit einem Patienten verbunden werden kann,
- ein zweites Endstück (17) mit einer Volumenverengung. die eine Mündung (15) bildet, die eine zylindrische oder kegelstumpfförmige Innenwand aufweist und -dazu konfiguriert ist, einen Luer- Stecker (20) aufzunehmen, und
- eine zentrale Kammer (13), die einerseits in den Anschluss (12) mündet und andererseits in die Mündung (15) und
- einem Schlüssel (21), der einen in der Kammer (13) angeordneten Federabschnitt (22) aufweist und einen Kopf (23), der in die Mündung (15) hineinragt,
- wobei der Federabschnitt (22) in der Kammer (13) zusammengedrückt werden soll
wenn ein Luer-Stecker (20) in die Öffnung (15) eindringt und gegen den Kopf (23) des Schlüssels (21) drückt, um eine hydraulische Verbindung zwischen dem Luer Stecker (20) und dem Anschluss (12) herzustellen,
***dadurch gekennzeichnet, dass*** diese Innenwand der Mündung (15) Haltemittel (25) mit einem vorspringenden Winkel aufweist, die radial in Richtung des Zentrums der Mündung (15) vorstehen, so dass sie in den Luer-Stecker (20) eindringen können, damit die durch den Druck eines Luer-Steckers (20) auf die Mündung (15) erzeugte Reibungskraft größer ist als die Rückstellkraft des Kopfes (23) des Schlüssels (21) in der Mündung (15).

2. Luer-Buchse (10) nach Anspruch 1, ***dadurch gekennzeichnet, dass*** die Mündung (15) und die Haltemittel (25) aus Polykarbonat bestehen.

3. Luer-Buchse (10) nach Anspruch 1 oder 2, ***dadurch gekennzeichnet, dass*** die Haltemittel (25) ein dreieckiges Profil aufweisen, eine erste, im Wesentlichen ebene Seite ist so konfiguriert, dass ein Luer-Stecker (20) in die Mündung (15) hinabgleiten kann und eine zweite, im Wesentlichen ebene Seite ist so konfiguriert, dass der Kopf (23) des Schlüssels (21) in der Mündung (15) hinaufgleiten kann.

4. Luer-Buchse (10) nach Anspruch 3, ***dadurch gekennzeichnet, dass*** die Haltemittel (25) ein dreieckiges, gleichschenkliges Profil aufweisen, die erste Seite hat eine größere Länge als die Länge der zweiten Seite.

5. Luer-Buchse (10) nach einem der Ansprüche 1 bis 4, ***dadurch gekennzeichnet, dass*** die Abdeckung (11), zwischen dem Volumen der Kammer (13) und dem der Mündung (15), eine Innenschulter (30) aufweist, die Haltemittel (25) sind in der Nähe der Schulter (30) angeordnet.

6. Luer-Buchse (10) nach einem der Ansprüche 1 bis 4, ***dadurch gekennzeichnet, dass*** die Haltemittel (25) eine Höhe zwischen 10 und 50 µm und eine Länge zwischen 30 und 80 µm aufweist.

7. Luer-Buchse (10) nach einem der Ansprüche 1 bis 6, ***dadurch gekennzeichnet, dass*** sie eine Nadel (32), angeordnet in der Kammer (13) enthält, deren Innenvolumen mit dem Anschluss (12) verbunden ist, die Nadel (32) ist dazu bestimmt, eine Mikro- Öffnung einer Abschlusswand (33) des Kopfes (23) des Schlüssels (21) zu durchdringen, wenn der Schlüssel (21) in der Kammer (13) durch einen Luer-Stecker (20) zusammengepresst wird.

8. Luer-Buchse (10) nach einem der Ansprüche 1 bis 7, ***dadurch gekennzeichnet, dass*** sie einen Ring (31) enthält, der um den Kopf (23) des Schlüssels (21) angeordnet ist und dazu bestimmt ist, die Verschiebung des Schlüssels (21) rechtwinklig entlang einer Länge der Abdeckung (11) zu führen.

## Claims

1. Female Luer connector (10) comprising:
- a cover (11) having:
- a first end (16) suitable for being connected to a patient,
- a second end (17) comprising a restriction in volume forming an opening (15) having a cylindrical or frustoconical inner wall and configured to receive a male luer connector (20), and
- a central chamber (13) leading on the one hand to the coupling (12) and on the other hand to the opening (15), and
- a seal member (21) with a portion forming a spring (22) arranged within the chamber (13) and a head (23) extending into the opening (15),
- the portion forming a spring (22) is designed to be compressed within the chamber (13) when a male Luer connector (20) penetrates the opening (15) and borders against the head (23) of the seal member (21) in order to establish a hydraulic connection between the male Luer connector (20) and the coupling (12),
***characterized in that*** the said inner wall of the opening (15) includes retaining means (25) having a projecting angle that extends radially towards the center of the opening (15) to penetrate a male luer connector (20) such that the frictional force generated by the male Luer connector (20) pressing against the opening (15) is greater than the spring force of the head (23) of the seal member (21) within the opening (15).

2. Female Luer connector (10) according to claim 1, ***characterized in that*** the opening (15) and the retaining means (25) are made of polycarbonate.

3. Female Luer connector (10) according to claim 1or 2, ***characterized in that*** the retaining means (25) have an isosceles triangular profile, a first significantly planar side is configured such as to allow a male Luer connector (20) to descend into the opening (15) and a second significantly planar side is configured to allow the head (23) of the seal member (21) to retract within the opening (15).

4. Female Luer connector (10) according to claim 3, ***characterized in that*** the retaining means (25) has a profile in the form of an isosceles triangle, the first side having a length exceeding a length of the second side.

5. Female Luer connector (10) according to any one of claims 1 to 4, ***characterized in that*** the cover (11) has an internal angled slope piece (30) between the volume of the chamber (13) and that of the opening (15), the retaining means (25) being arranged in close proximity to said slope (30).

6. Female Luer connector (10) according to any one of claims 1 to 4, ***characterized in that*** the retaining means (25) have a height of between 10 and 50 µm and a length of between 30 and 80 µm.

7. Female Luer connector (10) according to one of claims 1 to 6, ***characterized in that*** it comprises a needle (32) arranged within the chamber (13) whose internal volume is connected to the coupling (12), the needle (32) is designed to pass through a micro-opening of an end wall (33) of the head (23) of the seal member (21) when the seal member (21) is compressed within the chamber (13) by a male Luer connector (20).

8. Female Luer connector (10) according to one of claims 1 to 7, ***characterized in that*** it has a ring (31) arranged around the head (23) of the seal member (21) and designed to guide the movement of the seal member (21) in a straight manner along the length of the cover (11).
